Europäisches Patentamt

European Patent Office

Office européen des brevets

(11) Publication number: **0 168 689**
**B1**

(12) **EUROPEAN PATENT SPECIFICATION**

(45) Date of publication of patent specification: **26.09.90**

(21) Application number: **85107944.2**

(22) Date of filing: **28.06.85**

(51) Int. Cl.⁵: **G 01 N 33/576,**
**G 01 N 33/543**

(54) Immunoassay for detection of common determinant antibody to hepatitis B.

(30) Priority: **16.07.84 US 631032**

(43) Date of publication of application:
**22.01.86 Bulletin 86/04**

(45) Publication of the grant of the patent:
**26.09.90 Bulletin 90/39**

(84) Designated Contracting States:
**AT BE CH DE FR GB IT LI LU NL SE**

(56) References cited:
**US-A-4 038 378**

**Gastroenterology, vol 72, no.2, 1979 J.H. Hoofnagle et al. "Subtyping of Hepatitis B Surface Antigen and Antibody by Radioimmunoassay" pages 290-296**

(73) Proprietor: **ABBOTT LABORATORIES**
**14th Street and Sheridan Road North St**
**North Chicago Illinois 60064 (US)**

(72) Inventor: **Decker, Richard H.**
**924 Castlewood Lane**
**Deerfield Illinois (US)**
Inventor: **Gutierrez, Robin A.**
**34859 N. Hunt Club Road**
**Gurnee Illinois (US)**

(74) Representative: **Modiano, Guido et al**
**MODIANO & ASSOCIATI S.A.S. Via Meravigli, 16**
**I-20123 Milan (IT)**

Courier Press, Leamington Spa, England.

## Description

### Background of the invention

The sera of patients with acute viral hepatitis B infections (HBV) are characterized by the presence of immunochemically measurable amounts of hepatitis B surface antigen (HBsAg) from the surface of the virus. Some patients develop chronic hepatitis or a carrier state and maintain HBsAg in their serum, but the majority of patients recover. The sera of patients recovering from HBV infections are characterized by the disappearance of HBsAg and the appearance of antibody to hepatitis B surface antigen (anti-HBs). The presence of measurable anti-HBs in serum, then, is an indicator of convalescence and also immunity to subsequent reinfections from HBV.

There are several strains of HBsAg which are characterized by the antigenic determinants on their surfaces. The five main antigenic determinants are HBsAg/$a$, $d$, $y$, $w$ and $r$. The HBsAg/$a$ antigenic determinant is common to all strains of HBV, while $d$ and $y$ are exclusive of one another on the same HBV molecule, as are the $w$ and $r$ determinants. Therefore, there are four main antigenic subtypes of HBsAg which are $adw$, $ayw$, $adr$ and $ayr$. These antigens are responsible for eliciting humoral antibody responses to HBsAg, and anti-HBs antibodies are actually a number of antibodies to $a$, $d$, $y$ and, to a lesser extent, $w$ and $r$ antigenic determinants.

The major antibody responses elicited by exposure to HBsAg are production of antibodies to HBsAg/$a$, $d$ and $y$ (anti-HBs/$a$, $d$ and $y$). Since the $a$ antigenic determinant is found in all strains of HBV, anti-HBs/$a$ is believed to be the only antibody that provides complete immunity to all strains of HBV.

For purposes of the description herein, the main antigen subtypes of HBsAg are referred to as HBsAg/$ad$ and HBsAg/$ay$; however, it is to be understood that these subtypes carry the lesser antigenic determinants $r$ or $w$.

Recently, vaccines for HBV have been developed in order to produce immunity to the virus. Most of these vaccines are prepared from predominantly one subtype of HBsAg, i.e., either HBsAg/$ad$ or HBsAg/$ay$. A problem with these vaccines is that after vaccination, a great percentage of anti-HBs found in the sera of vaccinated patients is not anti-HBs/$a$, but rather anti-HBs/$d$ or anti-HBs/$y$, so that these immunized patients may still be susceptible to HBV. Therefore, a test that detects the presence of anti-HBs/$a$ in vaccinated patients or patients convalescing from an HBV infection is desirable in order to measure immunity to all HBsAg subtypes.

Most commercially available immunoassays for anti-HBs do not discriminate between the several antibody subtypes. Instead, these assays are direct assays in which antigen comprising HBsAg/$ad$ and HBsAg/$ay$ mixtures are immobilized or coated on a solid phase such as a polystyrene bead. Test samples are screened for the presence of anti-HBs by contacting the sample with the HBsAg-coated solid phase. Any of the anti-HBs subtypes, anti-HBs/$a$, $y$ or $d$, which may be present in the sample will react with the HBsAg-coated solid phase. Next, the solid phase is washed to remove unbound material, and labeled HBsAg comprising a mixture of HBsAg/$ad$ and HBsAg/$ay$ conjugated to an enzymatic, radioactive or fluorescent label, is added to the solid phase to form an antigen-antibody-antigen sandwich. The label is then measured as an indication of the amount of antibody present in the sample. Since both the HBsAg on the solid phase and the labeled HBsAg have multiple antigenic determinants, these assays detect any and all anti-HBs subtypes present in the test sample and cannot differentiate between them.

Hoofnagle, et al., *Gastroenterology*, 72:290-296 (1977), describe a method for detecting subtypes of HBsAg and anti-HBs by a neutralization procedure in which a test sample is mixed with different antigenic strains of HBsAg or anti-HBs before the sample is assayed by a conventional technique (Ausab® Radioimmunoassay, Abbott Laboratories, North Chicago, Illinois). The neutralization patterns of antigens and antibodies are then interpreted to determine the amounts of antigen or antibody subtypes in the test sample.

Legler, et al., *Develop. Biol. Std.*, 54:179—189 (1983), and Jilg, et al., *J. Med. Virol.*, 13:171—178 (1984), disclose yet another method for detecting subtypes of anti-HBs by screening samples with a series of solid phases coated with specific antigenic subtypes, HBsAg/$ad$ or HBsAg/$ay$, and comparing the potency of reaction with the different solid phase antigens. This method and the method described by Hoofnagle, et al., supra, require several test determinations for the same sample and a quantitative comparison between at least two test results.

### Definitions

As used herein, "test sample" refers to biological fluids including human biological fluids such as human serum, plasma or urine. The term "reagent(s)" as used herein refers to any components to be added in the steps of an immunoassay such as solid phase coated with an antigen or a labeled antigen.

The term "heterologous antigen subtypes" refers to different antigen subtype species which are mutually exclusive on the same molecule. Examples of heterologous antigen subtypes for hepatitis B surface antigen are HBsAg/$adw$ and HBsAg/$ayr$ as well as HBsAg/$adr$ and HBsAg/$ayw$.

### Summary of the invention

According to the invention, a direct immunoassay is provided which detects antibody to hepatitis B surface antigen/$a$ utilizing a single solid phase. This direct assay comprises immobilizing one specific antigen subtype of HBsAg selected from HBsAg/$adw$, HBsAg/$ayr$, HBsAg/$adr$ and HBsAg/$ayw$ on a solid phase such as a bead, test tube, microtiter plate, nitrocellulose sheet or derivatized paper. The solid phase is then reacted with a test sample such as human serum, plasma or urine containing an unknown amount of anti-HBs antibody. Next the solid phase is reacted with an *ayr*, *adw*, *ayw* or *adr* antigen subtype of HBsAg labeled with a suitable marker, the labeled antigen/*a* subtype being chosen according to said antigen subtype of HBsAg immobilized on the solid phase the said immobilized subtype being HBsAg/*adw*, HBsAg/*ayr*, HBsAg/*adr* or HBsAg/*ayw*, respectively. Examples of suitable markers include enzymes, radioisotopes, and other reagents which provide measurable colorimetric or fluorometric activity or radioactivity. The solid phase and unreacted reagents of the assay are separated, and the presence of the marker is measured in either the solid phase or the unreacted reagents. If an enzyme is utilized as the marker, a soluble substrate for the enzyme is added, and the enzyme's conversion from a colorless precursor to a colored product is measured spectrophotometrically.

Detailed description of the invention

The immunoassay of the invention can be performed in a two-step or one-step procedure. In the two-step procedure, the first step comprises incubating a solid phase coated with one HBsAg subtype selected from HBsAg/*adw*, HBsAg/*ayr*, HBsAg/*adr* and HBsAg/*ayw* with a test sample containing an unknown amount of anti-HBs/*a*. The solid phase is washed, and in the second step, the solid phase is incubated with an HBsAg subtype selected from HBsAg/*ayr*, HBsAg/*adw*, HBsAg/*ayw* and HBsAg/*adr* labeled with a suitable marker, the labeled antigen/*a* subtype being chosen according to said HBsAg subtype coated on the solid phase the said subtype coated on the solid phase being HBsAg/*adw*, HBsAg/*ayr*, HBsAg/*adr* and HBsAg/*ayw*, respectively. The solid phase and unreacted reagents are separated, and the presence of the marker is measured in either the solid phase or the unreacted reagents as a determination of the presence or amount of anti-HBs/*a* present in the sample.

The one-step procedure comprises simultaneously adding to a bead coated with one HBsAg subtype selected from HBsAg/*adw*, HBsAg/*ayr*, HBsAg/*adr* and HBsAg/*ayw*, a test sample and an *ayr*, *adw*, *ayw* or *adr* HBsAg subtype labeled with a suitable marker, the labeled antigen/*a* subtype being chosen according to said HBsAg subtype coated on the solid phase the said subtype coated on the solid phae being GBsAg/*adw*, HBsAg/*ayr*, HBsAg/*adr* or HBsAg/*ayw*, respectively. After one incubation period, the solid phase and unreacted reagents are separated and the marker is measured as previously described for the two-step procedure.

In this assay, only the common site between the antigenic subtype, i.e., HBsAg/*a* will react to form an antigen-antibody-antigen sandwich. The inventive assay does not detect the subtype antibodies anti-HBs/*d*, *y*, *w* or *r*.

Example 1

This example illustrates a two-step direct immunoassay for the detection of anti-HBs/*a* utilizing a radioactive label, carried out in accordance with the invention. The radioactively labeled antigen is prepared according to the method of Greenwood et al., *J. Biochem.*, 89:114—123 (1963).

A polystyrene bead (6.35 mm or 1/4 inch) is coated with HBsAg/*ayr* or *ayw* antigen at 2 μg/ml in .01M tris-hydrozymethyl-aminomethane ("Tris") buffer, pH 7.0, overnight at room temperature. Next 200 μl human serum sample containing an unknown amount of anti-HBs/*a* is added to the bead and incubated on a level surface for 2—12 hours at 25—45°C and preferably for 4 hours at 40°C. The bead is washed two times with 5 ml deionized water. 200 μl HBsAg/*adw* or *adr* antigen respectively, labeled with $^{125}$I, is added to the washed bead and incubated for 1—4 hours at 25—45°C and preferably for 2 hours at 40°C. The bead is washed a second time with 5 ml deionized water and transferred to a test tube for counting of radioactivity on a gamma scintillation counter such as an ANSR® gamma counter made by Abbott Laboratories, North Chicago, Illinois. The greater the amount of anti-HBs/*a* present in the serum sample, the greater the amount of radioactivity.

The above immunoassay can also be performed utilizing a polystyrene bead coated with HBsAg/*adr* or *adw* antigen. In this assay, an HBsAg/*ayw* or *ayr* antigen respectively, labeled with $^{125}$I, is used. Both assays perform equally well for the detection of anti-HBs/*a*, so long as the antigen subtype used to coat the bead and the antigen subtype of the labeled HBsAg are heterologous.

Example 2

This example is a two-step direct immunoassay for anti-HBs/*a* utilizing an enzyme. Horseradish peroxidase labeled antigen is prepared according to the method of Nakane et al., *J. Histochem. Cytochem.*, 22:1084—1091 (1974).

A polystyrene bead is coated with HBsAg/*ayr* or *ayw* antigen as described in Example 1. 200 μl human serum sample is added to the bead and incubated on a level surface for 2—12 hours at 25—45°C and preferably for 4 hours at 40°C. The bead is then washed two times with 5 ml deionized water. Next, 200 μl HBsAg/*adw* or *adr* antigen respectively, labeled with horseradish peroxidase, is added to the bead and incubated for 1—4 hours at 25—45°C and preferably for 2 hours at 40°C. The bead is washed two times with 5 ml deionized water, and the bead is transfered to a test tube for development of color. 300 μl o-phenylenediamine (OPD) substrate solution (o-phenylenediamine-2HCl, 12.8 mg tablet diluted in citrate-phosphate buffer containing .02% hydrogen peroxide) is added to the bead in the test tube and incubated for 30 minutes at room temperature. Next, 1.0 ml OSM sulfuric acid is added to the tube. Color produced is read on a spectrophotometer with absorbance determined at a wavelength of approximately 492 nm. The greater the amount of anti-HBs/*a* in the sample, the higher the absorbance.

As explained in Example 1, the procedure for Example 1 can be followed reversing the HBsAg subtype selected for the bead with the HBsAg subtype selected to be labeled, so long as one antigen is heterologous to the other.

Example 3

This example demonstrates a one-step assay according to the invention, utilizing either a radioactive or enzyme label.

A polystyrene bead is coated with HBsAg/*ayr* or *ayw* antigen as set forth in Example 1. 100 µl of human serum sample and 100 µl of HBsAg/*adw* or *adr* antigen respectively, labeled with either $^{125}$I or horseradish peroxidase, are added to the bead and incubated for 2—12 hours at 25—45°C and preferably for 4 hours at 40°C. The bead is then washed two times with 5 ml deionized water and transferred to a test tube for counting of radioactivity (Example 1) or development of color and measurement of absorbance (Example 2).

Example 4

This example illustrates a method of preparing HBsAg/*ad* (HBsAg/*adw* or adr) or HBsAg/*ay* (HBsAg/*ayw* or *ayr*) which is then used to coat a solid phase or conjugated to a marker such as a radioactive or enzymatic label.

HBsAg/*ad* and HBsAg/*ay* are obtained from sera or plasma units collected from human donors who are HBsAg carriers. The HBsAg subtypes of the individual donor sera or plasma units are determined as follows:

1. 200 µl HBsAg positive serum are added to two wells of a microtiter plate;

2. ONe anti-HBs coated bead (Ausria® Radioimmunoassay, Abbott Laboratories, North Chicago, Illinois) is added to each well;

3. The beads and sera are incubated at room temperature overnight and then washed two times with 5 ml deionized water;

4. 200 µl monospecific anti-*d* antibody is added to one bead and 200 µl monospecific anti-*y* antibody is added to the other bead and incubated for 1 hour at 45°C. The monospecific anti-*d* and anti-*y* are prepared according to the method of Ling et al., *Science*, 180: 203—205. The beads are then washed with 5 ml deionized water;

5. Beads are transferred to test tubes and radioactivity is counted on a gamma scintillation counter. HBsAg/*ad* sera will have elevated radioactivity in the anti-*d* test and lower radioactivity in the anti-*y* test. Conversely, HBsAg/*ay* sera will have elevated radioactivity in the anti-*y* test and lower radioactivity in the anti-*d* test.

Plasma or sera units which are positive for HBsAg/*ad* are pooled with other HBsAg/*ad* positive units, and HBsAg/*ay* positive units are pooled with other HBsAg/*ay* positive units. The pooled HBsAg/*ad* or HBsAg/*ay* sera or plasma are purified by alternate isopycnic banding and cesium chloride and sucrose density sedimentation according to the method of Gerin et al., *J. Virol.*, 4:763—768 (1969).

The HBsAg/*ad* and HBsAg/*ay* preparations are generally 95% pure as determined by quantitative immunodiffusion techniques which are well known to those skilled in the art.

There are many advantages to this *a*-specific, anti-HBs assay. First, the immunoassay is more sensitive and specific then previous anti-HBs assays which detect any and all subtype antibodies. Second, the assay detects only the common type antibody to HBV, anti-*a* which is believed to be the most protective antibody against infection or reinfection to HBV. Third, the assay is simple to perform.

**Claims**

1. An immunoassay for detecting antibody to hepatitis B surface antigen/*a* comprising the steps of:

   (a) immobilizing an *adw*, *ayr*, *adr* or *ayw* hepatitis B surface antigen/*a* subtype on a solid phase;

   (b) contacting the solid phase with a test sample;

   (c) contacting the solid phase with an *ayr*, *adw*, *ayw* or *adr* hepatitis B surface antigen/*a* subtype labeled with a marker, the labeled antigen/*a* subtype being chosen according to said antigen/*a* subtype immobilized on the solid phase, the said immobilized subtype being HBsAg/*adw*, HBSAg/ *ayr*, HBsAg/*adr* or HBsAg/*ayw*, respectively;

   (d) separating unreacted reagents from the solid phase; and

   (e) measuring the presence of the marker in the solid phase or in the unreacted reagents to detect the presence or amount of anti-hepatitis B surface antigen/*a* present in the sample.

2. The immunoassay of Claim 1 wherein the marker is an enzyme.

3. The immunoassay of Claim 1 wherein the marker is a radioisotope.

4. The immunoassay of Claim 1 wherein steps (b) and (c) are performed simultaneously.

5. A method for detecting antibody to hepatitis B surface antigen/*a* subtype comprising:

   (a) coating a polystyrene bead with an *adw*, *ayr*, *adr* or *ayw* subtype of hepatitis B surface antigen;

   (b) adding human serum sample to the coated bead;

   (c) incubating for 2—12 hours at 25—45°C;

   (d) washing the bead with deionized water;

   (e) adding to the bead an *ayr*, *adw*, *ayw* or *adr* subtype of hepatitis B surface antigen/*a* labeled with a marker, the labeled antigen/*a* subtype being chosen according to said antigen/*a* subtype coated on the bead, the said coated subtype being HBsAg/ *adw*, HBsAg/*ayr*, HBsAg/*adr* or HBsAg/*ayw*, respectively;

   (f) incubating for 1—4 hours at 25—45°C;

   (g) washing the bead with deionized water;

   (h) separating unreacted reagents from the bead; and

(i) measuring the presence of the marker in the unreacted reagents or on the bead.

6. THe method of Claim 5 wherein the marker is ¹²⁵I.

7. The method of Claim 5 wherein the marker is horseradish peroxidase.

8. The method of Claim 5 wherein step (c) comprises incubating for 4 hours at 40°C, and step (f) comprises incubating for 2 hours at 40°C.

**Patentansprüche**

1. Immunologischer Test zur Feststellung eines Antikörpers gegen Hepatitis B-Oberflächenantigen/a, umfassend die Stufen:

(a) Immobilisieren einer *adw-*, *ayr-*, *adr-* oder *ayw*-Untertype von Hepatitis B-Oberflächenantigen/a auf einer festen Phase;

(b) In-Berührung-Bringen der festen Phase mit einer Testprobe;

(c) In-Berührung-Bringen der festen Phase mit einer mit einem Marker markierten *ayr-*, *adw-*, *ayw-* oder *adr*-Untertype von Hepatitis B-Oberflächenantigen/a, wobei die markierte Antigen/a-Untertype in Abhängigkeit von der auf der festen Phase immobilisierten Antigen/a-Untertype gewählt wird, wobei es sich bei der genannten immobilisierten Untertype um HBsAg/*adw*, HBsAg/*ayr*, HBsAg/*adr* bzw. HBsAg/*ayw* handelt;

(d) Abtrennen nicht-umgesetzter Reagenzien von der festen Phase; und

(e) Messen des Vorliegens des Markers in der festen Phase oder in den nicht-umgesetzten Reagenzien zur Feststellung des Vorliegens oder der Menge von in der der Probe enthaltenem Anti-Hepatitis B-Oberflächenantigen/a.

2. Immunologischer Test nach Anspruch 1, worin der Marker ein Enzym ist.

3. Immunologischer Test nach Anspruch 1, worin der Marker ein Radioisotop ist.

4. Immunologischer Test nach Anspruch 1, worin die Stufen (b) und (c) gleichzeitig ausgeführt werden.

5. Verfahren zur Feststellung von Antikörper gegen eine Hepatitis B-Oberflächenantigen/a-Untertype, umfassend:

(a) Überziehen einer Polystrolperle mit einer *adw-*, *ayr-*, *adr-* oder *ayw*-Untertype von Hepatitis B-Oberflächenantigen;

(b) Zusetzen einer Humanserumprobe zu der überzogenen Perle;

(c) Inkubieren während 2—12 Studen bei 25—45°C C;

(d) Waschen der Perle mit entionisiertem Wasser;

(e) Zusetzen einer mit einem Marker markierten *ayr-*, *adw-*, *ayw-* oder *adr*-Untertype von Hepatitis B-Oberflächenantigen/a zu der Perle, wobei die markierte Antigen/a-Untertype in Abhängigkeit von der als Überzug auf die Perle aufgebrachten Antigen/a-Untertype ausgewählt wird, wobei die genannte aufgebrachte Untertype HBsAg/*adw*, HBsAg/*ayr*, HBsAg/*adr* bzw. HBsAg/*ayw* ist;

(f) Inkubieren während 1—4 Stunden bei 25—45° C;

(g) Waschen der Perle mit entionisiertem Wasser;

(h) Abtrennen nicht-umgesetzter Reagenzien von der Perle; und

(i) Messen des Vorliegens des Markers in den nicht-umgesetzten Reagenzien oder auf der Perle.

6. Verfahren nach Anspruch 5, worin der Marker ¹²⁵I ist.

7. Verfahren nach Anspruch 5, worin der Marker Meerrettichperoxidase ist.

8. Verfahren nach Anspruch 5, worin die Stufe (c) ein Inkubieren während 4 Stunden bei 40°C umfaßt und die Stufe (f) ein Inkubieren während 2 Stunden bei 40°C umfaßt.

**Revendications**

1. Un procédé de dosage immunologique pour détecter l'anti-corps de l'antigène de surface de l'hépatite B/a comprenant les étapes suivantes;

(a) immobilisation sur une phase solide d'un sous-type d'antigène de surface de l'hépatite B/a, *adw*, *ayr*, *adr* ou *ayw*;

(b) mise en contact de la phase solide avec un échantillon d'essai;

(c) mise en contact de la phase solide avec un sous-type d'antigène de surface de l'hépatite B/a, *ayr*, *adw*, *ayw* ou *adr* marqué par un marqueur, le sous-type d'antigène/a marqué étant choisi conformément audit sous-type d'antigène/a immobilisé sur la phase solide, ledit sous-type immobilisé étant la AgHBs/*adw*, le AgHBs/*ayr*, le AgHBs/*adr* ou le AgHBs/*ayw*, respectivement;

(d) séparation des réactifs n'ayant pas réagi de la phase solide; et

(e) measure de la présence du marqueur dans le phase solide ou dans les réactifs n'ayant par réagi pour détecter la présence ou la quantité d'antigène de surface de l'anti-hépatite B/a présent dans l'échantillon.

2. Le procédé de dosage immunologique selon la revendication 1, selon lequel le marqueur est une enzyme.

3. Le procédé de dosage immunologique selon la revendication 1, selon lequel le marqueur est un radioisotope.

4. Le procédé de dosage immunologique selon la revendication 1, selon lequel les étapes (b) et (c) sont conduites simultanément.

5. Un procéde pour détecter l'anticorps du sous-type d'antigène de surface de l'hépatite B/a comprenant:

(a) l'enrobage d'une perle de polystyrène par un soustype *adw*, *ayr*, *adr* ou *ayw* de l'antigène de surface de l'hépatite B;

(b) l'addition d'un échantillon de sérum humain à la perle enrobée;

(c) l'incubation pendant 2—12 h à 25—45°C;

(d) le lavage de la perle par de l'eau désionisée;

(e) l'addition à la perle d'un sous-type *ayr*, *adw*, *ayw* ou *adr* de l'antigéne de surface de l'hépatite B/a marqué par un marqueur, le sous-type d'antigène/a marqué étant choisi selon ledit sous-type d'antigène/a enrobant la perle, ledit sous-type d'enrobage étant le AgHBs/*adw*, le AgHBs/*ayr*, le AgHBs/*adr* ou le AgHBs/*ayw*, respectivement;

(f) l'incubation pendant 1—4 h à 25—45°C;

(g) le lavage de la perle par de l'eau désionisée;

(h) la séparation des réactifs n'ayant pas réagi de la perle; et

(i) la mesure de la présence du marqueur dans les réactifs n'ayant pas réagi ou sur la perle.

6. Le procédé selon la revendication 5, selon lequel le marqueur est le $^{125}$I.

7. Le procédé selon la revendication 5, selon lequel le marqueur est la peroxydase de raifort.

8. Le procédé selon la revendication 5, selon lequel l'étape (c) comprend l'incubation pendant 4 h à 40°C, et l'étape (f) comprend l'incubation pendant 2 h à 40°C.